# EUROPEAN PATENT APPLICATION

(11) **EP 4 703 045 A1**
(43) Date of publication of application: **04.03.2026**
(21) Application number: 24198246.1
(22) Date of filing: 03.09.2024
(51) Int. Cl.: B05B 11/00, B05B 11/10

(54) **DISCHARGE HEAD AND FLUID DISPENSER WITH SUCH A DISCHARGE HEAD**

(71) Applicant: Aptar Radolfzell GmbH, 78315 Radolfzell (DE)
(72) Inventor: Girlanda, Lorenzo, 22020 Faloppio (IT); Corongiu, Marco, 6900 Massagno (CH); Aletti, Fabio, 21100 Varese (IT)
(74) Representative: Witte, Weller und Partner Patentanwälte mbB Stuttgart

(57) **Abstract**

The invention relates to a discharge head (10) for dispensing liquids, particularly pharmaceutical liquids. This discharge head (10) is designed to be attached to a liquid reservoir (100) and features a discharge head base (20) with a coupling device (22) for connecting to the liquid reservoir (100). It includes a discharge opening (86) and a pump device (40) for conveying liquid from the liquid reservoir (100) to the discharge opening (86), wherein the pump device (40) comprises a pump cylinder and a pump piston (32) that is at least temporarily guided in a sealing manner along a cylinder sliding surface (54).

A design is proposed where the pump piston (32) is fixedly arranged relative to the discharge head base (20), and the pump cylinder is formed by a cylinder component (50) that is movable relative to the discharge head base (20). The discharge head is equipped with a return spring (90) designed as a plastic bellows spring, which exerts a counteracting return force on the cylinder component (50) when it is moved in an actuating direction (2A) relative to the pump piston (32).

## Description

### APPLICATION FIELD AND PRIOR ART

The invention relates to a discharge head for dispensing liquids, particularly a discharge head for dispensing pharmaceutical liquids. Primarily, the invention is directed towards discharge heads intended for the nasal application of pharmaceutical liquids. The liquid is stored in a liquid reservoir until dispensing and can be discharged from it using the discharge head.

Such discharge heads are equipped with a pump device with a pump chamber that can be reduced in size by actuating the discharge head. By reducing the size of the pump chamber, the liquid contained therein is pressurized and discharged through a discharge opening, for example, through a discharge opening at the end of a nasal applicator into a nostril.

Such discharge heads are widely known. They usually have a pump device that includes a movable piston, which can be displaced along a cylinder fixed to the liquid reservoir by pressing down a finger rest associated to an applicator, thereby discharging the liquid from the pump chamber formed by the cylinder and piston.

From the prior art, such as DE 10 2011 082 420 B4, discharge heads are also known in which the pump piston of the pump device is fixed relative to a base of the discharge head. In such discharge heads, actuating the discharge head displaces the cylinder surface of a pump piston relative to the fixed pump piston to reduce the volume of the pump chamber.

Discharge heads are typically disposable products and are discarded as a whole after the liquid reservoir is emptied. Therefore, it is desirable to simplify the recycling process for such discharge heads as much as possible.

It is already known from the prior art to use plastic springs instead of metal springs to improve recyclability. However, plastics have a greater tendency to relaxation than metals. The restoring force generated by a plastic spring thus decreases over time, which can make it difficult to maintain a specified liquid quantity during dispensing or even hinder the operation of a liquid dispenser.

### OBJECT AND SOLUTION

The object of the invention is to provide a discharge head that combines good recyclability with high dosing accuracy and reliability.

According to the invention, a discharge head for dispensing liquids and a liquid dispenser with such a discharge head are proposed. As mentioned, such a discharge head and liquid dispenser are particularly intended for dispensing pharmaceutical liquids and especially for nasal liquids. The discharge head can be designed for dispensing liquid in the form of a non-atomized jet or a spray jet. In principle, it is also possible to use it as a discharge head for delivering individual drops.

The discharge head is designed to be attached to a liquid reservoir and has a discharge head base with a coupling device for connecting to the liquid reservoir. In particular, the coupling device can consist of an external thread or an inward-facing snap edge to allow the discharge head to be screwed or snapped onto the liquid dispenser. A dip tube may be provided integrally or as a separate component on the discharge head base, extending into the liquid reservoir.

As usual for such discharge heads, the discharge head has a discharge opening and a pump device for conveying liquid from the liquid reservoir to the discharge opening. In particular, the discharge opening may be provided at the distal end of a slim, elongated nasal applicator. Preferably, the discharge head is designed for dispensing the liquid in atomized form and has a swirl chamber upstream of the discharge opening, through which the pressurized liquid is set into rotation, causing it to break up into small droplets upon discharge.

The pump piston, which has a circumferential outer side that, at least temporarily during actuation, seals against an inner cylinder surface of the pump cylinder, is fixedly arranged relative to the discharge head base in a dispenser according to the invention. A movable pump cylinder is provided. For this purpose, a cylinder component that is movable relative to the discharge head base is provided, the inner side of which forms the mentioned cylinder surface.

To prepare the discharge head for a subsequent dispensing operation by increasing the pump chamber and drawing liquid from the liquid dispenser after the pump cylinder has been manually displaced and the pump chamber reduced in size, a return spring is provided. This spring counteracts the user's actuating force and enlarges the pump chamber at the end of a dispensing operation.

This return spring is designed as a plastic bellows spring, which, when the cylinder component is displaced in an actuating direction relative to the pump piston, exerts a counteracting return force on the cylinder component. As soon as the actuating force applied by the user ends, this plastic bellows spring pushes the cylinder component back towards its starting position, thereby drawing more liquid into the pump chamber.

It has been found that the combination of a pump piston fixed to the discharge head base and a plastic bellows spring offers particular advantages. With such a design featuring a fixed piston, it is particularly easy to achieve a consistent liquid discharge quantity in a manner that will be explained later. It also allows for an especially advantageous arrangement of the return spring. Preferably, the fixed pump piston is arranged at a distal end of a central tube, which extends from the discharge head base to the pump piston. This central tube may be surrounded by the plastic bellows spring, so that the central tube also acts as kink protection for the bellows.

Preferably, the plastic bellows spring has an expanding shape in one direction. The two opposite ends of the plastic bellows spring therefore have different diameters. In particular, the plastic bellows spring may have bellows windings that, starting from a wide end of the plastic bellows spring, decrease in diametertowards a narrow end of the plastic bellows spring. The term bellows windings here also refer to bellows windings that are connected in a spiral-like manner.

A plastic bellows spring with an expanding shape allows the spring force characteristic to be influenced. In particular, this can achieve a non-linear spring characteristic with progressive stiffness during compression. This may be desirable, as it is perceived as pleasant by the user in terms of haptics.

The wide end of the bellows spring with a larger diameter preferably forms a base end and is supported, at least indirectly, on the discharge head base. The narrow end of the bellows spring forms an applicator end and is supported, at least indirectly, on the cylinder component.

Preferably, the plastic bellows spring is designed as an internal component and is not visible to a user of the discharge head. For this purpose, it is particularly preferably arranged in an annular interior space, which is bounded at the bottom by the discharge head base, on the inside by the mentioned central tube, and at the top and outside by a depressible outer component.

The expanding shape and arrangement are also advantageous because a discharge head according to the invention preferably has an expanding shape from the applicator end towards the discharge head base, thus making particularly good use of the available space when a lower end of the bellows spring is expanded.

Particularly preferred is a design in which the discharge head has an actuating surface, with the wide end of the bellows spring extending into a receiving space located below the actuating surface relative to an actuating direction. In a radial direction, the lower end of the bellows spring preferably exceeds the outer diameter of the applicator, which is preferably designed as a slim, elongated nasal applicator. The outer diameter of the applicator is therefore preferably smaller tha n the outer diameter of the bellows spring at its lower end.

An interior space of the bellows spring can be connected to the environment outside the bellows spring. However, it may also be advantageous if the interior space of the bellows spring is isolated from the environment, so that the air contained therein is compressed when the bellows spring shortens and thereby contributes to the return force of the bellows spring.

Preferably, the pump device is designed in such a way that, starting from an unactuated position and thus a comparatively relaxed bellows spring, the liquid in the pump chamber is not immediately pressurized upon actuation. Instead, the pump device is preferably designed to leave the pump chamber initially in communicating connection with the liquid reservoir until an initial idle path of the cylinder component has been traversed. Only then is the pump chamber separated from an inflow channel, and pressurization and liquid discharge can begin.

Such a design is also referred to as a "lost motion" design. It is advantageous because the bellows spring gradually loses its restoring ability during extended storage and therefore may not fully reach an unactuated end position. However, due to the mentioned idle path, this does not affect dosing accuracy. The distance over which the pump piston and the cylinder component are displaced under pressurization of the liquid in the pump chamber is not negatively affected by the reduced restoring ability of the spring.

Preferably, the pump device is designed such that, starting from an unactuated end position, at least 10% of the displacement distance at the beginning of actuation constitutes the idle path. Particularly preferably, the proportion of the idle path in the total displacement distance is at least 12%, preferably even at least 14%.

A particularly advantageous design provides that the inner side of the cylinder component has at least one section where there is no circumferential seal between the pump piston and the innerside of the cylinder component, with the pump piston being arranged in this non-sealing section when the discharge head is in an unactuated state. Technically, the mentioned section can be realized, for example, by having the inner diameter of the cylinder component be circumferentially larger than the outer diameter of the pump piston. Alternatively, this circumferential interruption of the seal can also be achieved by axial grooves in the inner wall of the cylinder component.

The pump device has an outlet valve on the output side of the pump chamber, which preferably opens depending on pressure and closes during the return movement of the discharge head, so that new liquid can be drawn into the pump chamber from the liquid reservoir.

To realize this outlet valve, or in addition to such a pump outlet valve, an outlet valve is preferably provided, which is carried by the cylinder component.

A preferred design is one in which an outer component of the discharge head forms an applicator, particularly a slim nasal applicator. The outer component preferably additionally integrally forms an actuating surface for manual operation. In particular, this may be a circumferential actuating surface that surrounds the slim nasal applicator. The cylinder component is inserted into an inner cavity of the nasal applicator.

A valve body of the outlet valve formed by the cylinder component is provided at the distal end of the cylinder component and has a valve body that seals with a valve surface provided on the inner side of the outer component in the closed state. This valve body may be movable relative to the cylinder component in a manner to be explained further below, or remain fixed relative to it.

Guiding surfaces for guiding the valve body are preferably provided on the inner side of the outer component, which forms the outer shape of the applicator. In particular, ribs can be provided here to provide proper guidance of the valve body.

There are two fundamentally different designs possible regarding the coupling of the cylinder component with the outer component.

In a first design, it is provided that the outer component and the cylinder component are immovable relative to each other. This can be achieved by having the outer component and the cylinder component directly fixed to each other, for example, by a snap connection. It can also be achieved by having the cylinder component permanently pressed into an end position relative to the outer component by the bellows spring.

In such a case, the valve body of the outlet valve, which is upstream of the discharge opening, is movable relative to the cylinder component. In particular, it may be provided that an elastically deformable valve membrane is attached or provided at the distal end of the cylinder component, which supports the valve body and which, when pressurized in a valve chamber, forces the valve body into an open valve position. The valve body can, in particular, be integrally connected to the valve membrane. The membrane is preferably made of the same material as the bellows spring.

Preferably, it is provided that the outlet valve has a sealed counter chamber on the side of the valve membrane opposite the valve chamber, which is at least partially bounded by the cylinder component. This counter chamber is preferably isolated from a surrounding atmosphere.

Particularly preferably, this counter chamber is also the location where a limiter surface is provided to limit the displacement of the valve body from the closed valve position to the open valve position. If the outlet valve is opened under the influence of liquid pressure in the pump chamber and the valve chamber, the limiter surface limits the movement of the valve body and thus the maximum opening. It therefore prevents excessive discharge.

Preferably, a limiting pin extending towards the discharge opening is provided centrally at the distal end of the cylinder component, whose end face forms the mentioned limiter surface.

The valve component, which is provided at the distal end of the cylinder component and which preferably also integrally forms the valve body, preferably has an outer sealing collar, by means of which it is fitted onto a mounting surface of the cylinder component. In particular, the cylinder component may have a ring-shaped cylindrical outer surface onto which the sealing collar is fitted.

Preferably, the valve component has outwardly extending centering fins, by means of which it is guided in the outer component during assembly, so that it remains in its intended position when the cylinder component is inserted into the outer component.

The valve membrane can, in the simplest case, be a largely flat membrane that is externally attached to the cylinder component and centrally provided with the valve body. However, a design of the valve membrane in which a cross-section through the valve membrane between the sealing collar and the valve body shows at least two directional changes, with the cross-section of the valve membrane preferably having the shape of an elongated "S", is considered particularly advantageous. This shape promotes secure closing of the valve as well as secure opening. The twice-changing direction of the valve membrane allows the valve to be opened primarily through a bending deformation.

In the second general design for coupling the cylinder component with the outer component, it is provided that the cylinder component is designed to be movable relative to the outer component.

This allows the outlet valve to be opened and closed by the relative movement of the cylinder component and the outer component. This generally allows the mentioned return bellows spring to perform two functions: on the one hand, the return of the cylinder component relative to the discharge head base and the pump piston, and on the other hand, the function of a valve spring, by means of which a valve body is pressed towards its closed position.

In such a design, the valve body can be an integral part of the cylinder component and fixed relative to other parts of the cylinder component and in particular its cylinder sliding surface.

An alternative is to provide a limited movability of the valve body relative to the cylinder sliding surface, despite the overall movability of the cylinder component relative to the outer component.

Similar to the outlet valve described above, which was proposed for a fixed configuration of the cylinder component relative to the outer component, it is also provided in this case that a spring section, preferably in the form of a membrane, particularly bell-shaped, is provided between the distal end of the cylinder component and the valve body. A special design provides that the membrane, and preferably also the valve body, are integrally formed parts of the cylinder component, i.e., integrally formed with the cylinder sliding surface of the cylinder component. In this case, the membrane preferably has a significantly reduced wall thickness compared to the wall thickness of the cylinder component in the area of the cylinder sliding surface.

The movability of the valve body relative to the cylinder component is advantageous because it counteracts damage to the valve body by the restoring force of the return spring. If the restoring force of the return spring is still quite high in a relatively new discharge head, a valve body fixed relative to the cylinder component is pressed against the inner side of the outer housing with a high force. The movability of the valve body relative to the cylinder component allows this force to be somewhat cushioned, thereby avoiding damage to the surface of the valve body.

In a design with a cylinder component movable relative to the outer component, it is preferably provided that the plastic bellows spring is supported on the cylinder component and acts indirectly on the outer component. If the outer component is pressed down, it initially also presses the cylinder component down. As soon as this causes pressure to build up in the pump chamber and thus also in the valve chamber, the cylinder component is displaced to a greater extent than the outer component, causing the outlet valve to open.

In a specific design, it is provided that a spring element is provided, which directly applies a closing force to the outer component and the cylinder component, acting in the direction of displacing the valve body into a closed valve position.

In such a design, it is provided that not only or not exclusively the plastic bellows spring between the discharge head base and the cylinder component provides a closing force acting on the valve body, but an additional spring element is provided, which directly provides a closing force. Such an additional spring element can be useful to ensure quick closing of the outlet valve at the end of actuation. If the plastic bellows spring between the discharge head base and the cylinder component is weakened due to relaxation, quick closing of the valve and thus reliable suction of liquid for the next discharge is still ensured.

The additional spring element between the cylinder component and the outer component can be designed in various ways. In particular, a spring washer may be considered here, which is externally supported on the outer component and internally fixed to the cylinder component. The spring washer can also be an integrally molded section of the cylinder component. An alternative is a spring element in the form of a bellows. This can also be an integrally molded section of the cylinder component.

In the case of an integrally molded design, it should be noted that the cylinder sliding surface must be sufficiently dimensionally stable to allow smooth sliding of the pump piston. At the same time, the integrally molded spring element must allow sufficient deformation to open the outlet valve. It is preferred that the spring element has a wall thickness that is at most 50%, and preferably at most 20%, of the wall thickness of the cylinder component in the area of the cylinder sliding surface.

The discharge head preferably consists exclusively of plastic parts. Preferably, only plastics are selected that can be processed in a common recycling process. In particular, all components can be made from one of the plastics polyethylene (PE), polypropylene (PP), and polyethylene terephthalate (PET). However, it is particularly preferred if only one of these plastics is used for all components, particularly polyethylene (PE). Different grades of PE can be used for the various components. In particular, the plastic bellows spring, which acts as a return spring, and/or a deformable part of the outlet valve can be made of LDPE. It is also possible to manufacture the entire cylinder component from LDPE and achieve the required dimensional stability in the area of the cylinder sliding surface by a relatively large wall thickness.

A discharge head according to the invention combines good recyclability with high dosing accuracy. Furthermore, the design allows the discharge head to be constructed from relatively few components. Disregarding a possibly present bottle seal, a possibly separately formed dip tube, and a removable protective cap, the discharge head itself can be formed from only four parts, namely the outer component, the cylinder component, the plastic bellows spring, and the discharge head base. A particularly preferred design includes another elastic component and thus five parts (excluding the dip tube and bottle seal), which provides a spring membrane that allows for the movable attachment of the valve body to the cylinder component.

The invention relates not only to the discharge head itself but also to a liquid dispenser with such a discharge head. This liquid dispenser has a liquid reservoir, which is preferably made of plastic and particularly preferably made of PE. The liquid reservoir and the discharge head are matched to each other in such a way that the discharge head can be attached to a storage opening, particularly to a bottle neck of the liquid reservoir, particularly by a snap-fit.

In the filled state, the liquid reservoir is filled with the liquid to be dispensed, particularly a pharmaceutical liquid such as an aqueous solution containing active ingredients like decongestants (e.g., xylometazoline, oxymetazoline), antihistamines, corticosteroids (e.g., fluticasone, mometasone), or antibiotics.

Preferably, the liquid reservoir is a non-vented liquid reservoir. The discharge of liquid therefore creates a vacuum in the liquid reservoir, which must be overcome by the return spring when displacing the cylinder component to its unactuated starting position. However, designs with bottle venting are also possible, preferably with a filter for removing contaminants from the incoming air.

### BRIEF DESCRIPTION OF THE DRAWINGS

Further advantages and aspects of the invention will become apparent from the claims and the following description of preferred embodiments of the invention, which are explained below with reference to the figures.
Figures 1 and 2 show a first embodiment of a liquid dispenser and discharge head according to the invention.
Figure 3 shows an alternative design with a special inlet valve design.
Figures 4 and 5 illustrate the design of the outlet valve of the discharge head.
Figures 6 to 8 show further embodiments of a discharge head according to the invention.

### DETAILED DESCRIPTION OFTHE EMBODIMENTS

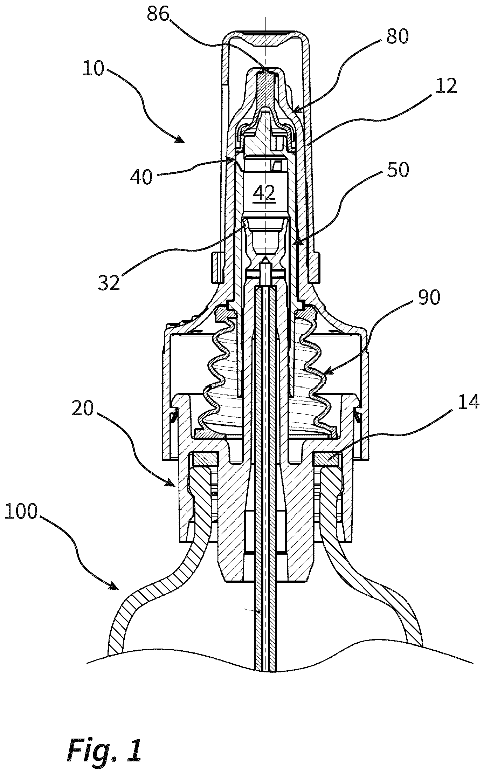

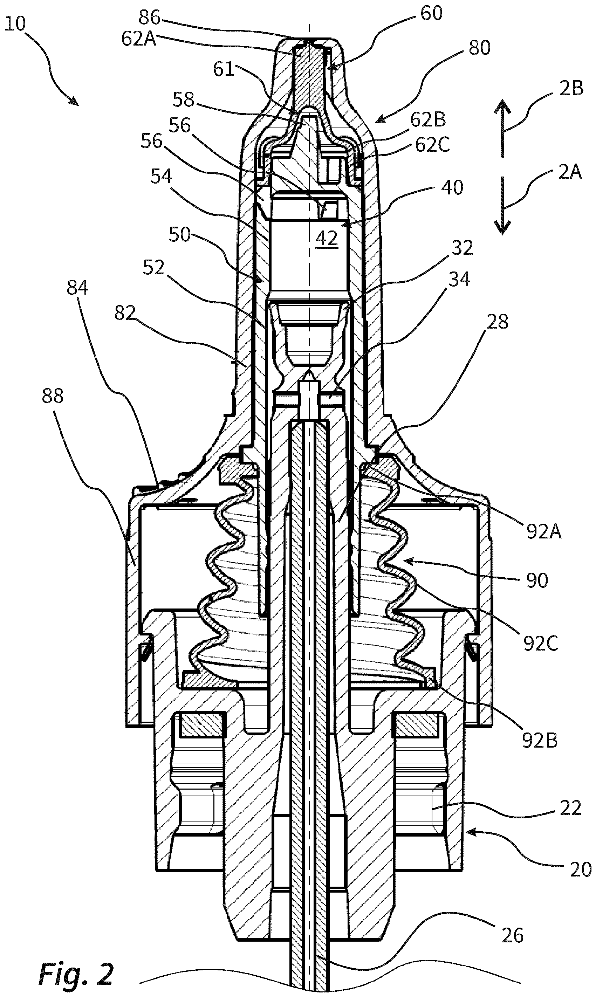

Figure 1 shows a first embodiment of a liquid dispenser according to the invention. This liquid dispenser has a bottle-like liquid reservoir 100, the bottle neck 102 of which has a geometry that allows a discharge head 10 to be snap-fitted. The discharge head 10 is shown in more detail in Figure 2.

The discharge head 10 has a discharge head base 20, on which a snap geometry is provided as a coupling device 22. With the aid of a ring-shaped bottle seal 14, the discharge head base 20 is sealed circumferentially against the bottle neck 102, so that the only connection from the liquid reservoir 100 to the outside is through a dip tube 26, which is inserted snugly into the discharge head base 20.

A movable outer component 80 can be depressed relative to the discharge head base 20 in an actuating direction 2A to cause the liquid to be discharged. The outer component 80 forms a slim, elongated nasal applicator 82, at the distal end of which a discharge opening 86 is provided. The outer component 80 transitions into an outward-facing collar, which forms an actuating surface 84. This is followed by a cylindrical outer surface 88, which is provided on the inside with holding contours, which correspondingly engage with holding contours on the discharge head base 20. These holding contours prevent the outer component 80 from being pulled off the discharge head base 20 and additionally serve as stops that define the end of movement during a return movement of the outer component 80 in a return direction 2B.

The discharge head 10 has a pump device 40 for conveying liquid from the liquid reservoir 100 to the discharge opening 86. This pump device 40 is a piston pump device and includes, for this purpose, a pump piston 32 that can be displaced in a cylinder.

The pump piston 32 is fixed and is integrally formed with the discharge head base 20 in this case. For this purpose, a central tube 28 extends upward from the discharge head base 20, within which liquid can flow towards the pump piston 32. Below the pump piston, radial bores 34 are provided through which the liquid exits the central tube 28 to then flow past the pump piston 32 into the pump chamber 42.

The pump piston 32 is arranged fixed relative to the discharge head base 20 due to this design and is therefore immovable relative to the discharge head base 20. The reduction of the volume of a pump chamber 42 thus occurs through relative displacement of the cylinder relative to the pump piston 32. For this purpose, a cylinder component 50 is provided, which is inserted into a cavity of the outer component and remains fixed in operation. When the entire outer component 80 is depressed by pressing the actuating surfaces 84, the cylinder component 50 is depressed to the same extent.

This results in a reduction of the volume of the pump chamber 42 and thus in the pressurization of the liquid contained therein, as will be described in more detail below.

To be able to discharge the liquid in the desired form, the discharge head 10 has an outlet valve 60. This outlet valve is formed by a movable valve body 62A, which, in the closed state, rests against an inner ring surface upstream of the discharge opening 86. The valve body 62A is part of an integral valve component 61, which is attached to a distal upper end of the cylinder component 50 by means of a valve membrane 62B. The valve membrane 62B is approximately bell-shaped. Its outer edge is sealingly attached to the cylinder component 50, so that a valve chamber 64 and a counter chamber 66 that does not come into contact with liquid are formed in isolation from each other.

A limiting pin 58 is provided at the distal end of the cylinder component 50 in the counter chamber 66, whose end face forms a limiter surface that limits the displacement of the valve body 62A. As the pressure in the pump chamber 42 and in the valve chamber 64 increases, the valve body is pressed downward against the force of the elastically deformed valve membrane 62B relative to the cylinder component 50 and the outer component 80, and the outlet valve 60 opens. The valve body 62A is guided by guide surfaces 89 on ribs of the outer component 80, so that there is no risk of jamming, even if the valve body 62A is integrally formed with the valve membrane 62B and for this purpose is made of a relatively soft plastic.

When the outlet valve 60 is open, the liquid is discharged through the discharge opening 86. In this case, a swirl chamber is provided upstream of the discharge opening 86, so that the liquid is discharged as a spray jet.

In the discharge head 10, a return spring 90 designed as a plastic bellows spring serves as a return spring. This return spring 90 has an expanding shape downwards, as the windings 92C of the plastic bellows spring have a larger diameter downwards. The wide lower end 92B of the return spring 90 is supported on a support surface surrounding the central tube 28 of the discharge head base 20. The opposite upper end 92A is supported from below on the cylinder component 50 or on the inner side of the outer component 80. Preferably, the end 92A is supported on the cylinder component 50, so that it is forcefully pressed into the outer component 80, eliminating the need for further fixation of the cylinder component 50 to the outer component 80.

The expanding shape of the return spring 90 causes an advantageous spring behavior. A shortening of the spring, starting from the unactuated position of Figures 1 and 2, initially occurs against a comparatively low spring force due to the large diameter at the lower end of the return spring 90. This facilitates actuation. The spring force increases significantly only as actuation progresses.

Furthermore, the expansion is advantageous because bellows springs with a larger diameter generally tend to relax less.

The return spring 90 makes good use of an interior space primarily formed by the discharge head base 20 and the outer component 80. The lower end 92B is expanded to the extent that it is located below the actuating surface. The upper end 92A is narrowed to the extent that it can rest on the comparatively slim cylinder component 50.

Plastic springs are generally subject to the phenomenon of relaxation. This means that plastic springs relax overtime under constant stress, and thus their spring characteristic changes. Although the design with an expanding bellows spring counteracts this effect, some degree of relaxation still occurs.

The pump device 40 is specially designed to counteract this effect by being configured to initially not pressurize the liquid in the pump chamber 42 upon actuation, starting from the unactuated end position of Figures 1 and 2, and only after traversing a predetermined idle path of the cylinder component 50 relative to the pump piston 32 does it begin to convey liquid. If the return spring 90 is no longer able, due to relaxation, to press the cylinder component 50 and the outer component 80 to the position of Figures 1 and 2 after actuation, this does not affect the dosing accuracy of the discharge head.

The idle path is structurally implemented in the design of Figures 1 and 2 by having a stepped inner surface of the cylinder component 50. A lower section 52 has a cross-sectional shape or diameter that does not allow the pump piston 32 to seal tightly. As long as the pump piston 32 moves in this section 52, the pump chamber 42 is not yet isolated from the liquid reservoir 100, as the liquid can flow past the pump piston 32 and through the radial bores 34 back into the liquid reservoir.

A subsequent section forms the cylinder sliding surface 54. Only when the cylinder component 50 has been depressed to the extent that the pump piston 32 seals circumferentially against the cylinder sliding surface 54 does pressurization of the liquid in the pump chamber 42 begin, which propagates through openings 56 in the cylinder component into the valve chamber 64 and causes the outlet valve 60 to open.

When the discharge is complete, i.e., when the cylinder component 50 has reached its lower end position and the pump piston 32 rests against the upper end of the pump chamber 42, the user releases the applied force, so that the outer component 80 and the cylinder component 50 rise again relative to the pump piston 32 under the action of the return spring 90. A vacuum is created in the pump chamber 42 and the valve chamber 64, ensuring that the outlet valve 60 closes quickly and securely. The pump piston 32 is displaced downwards along the cylinder sliding surface 54 against the force of the vacuum in the pump chamber 42. Only when the pump piston 32 separates from the cylinder sliding surface 54 can liquid flow into the pump chamber 42.

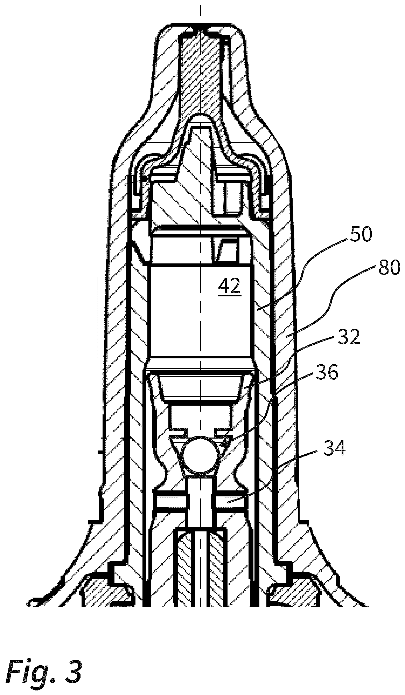

To facilitate the return stroke, an inlet valve 36 can be provided in the manner shown in Figure 3. This immediately opens during the return stroke, resulting in the suction of liquid into the pump chamber 42 during the return stroke.

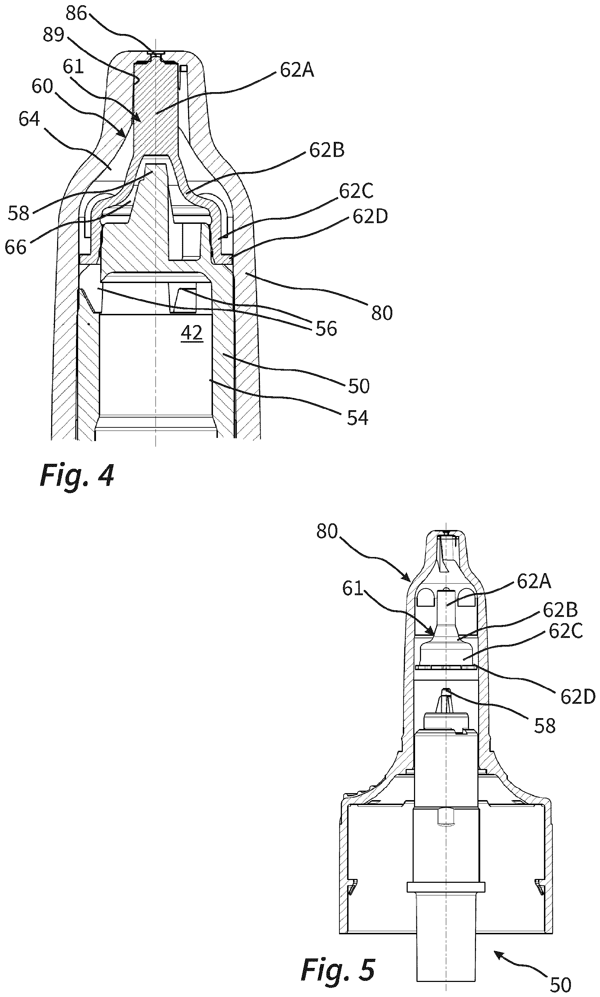

Figures 4 and 5 further illustrate the structure of the outlet valve 60. As can be seen, the valve component 61 is provided with centering fins 62D at the lower end in the area of a sealing collar 62C, which ensure centering of the valve component 61 during assembly. The limiting pin 58 and the ribs forming guide surfaces 89 for the valve body 62A can also be seen in Figure 5.

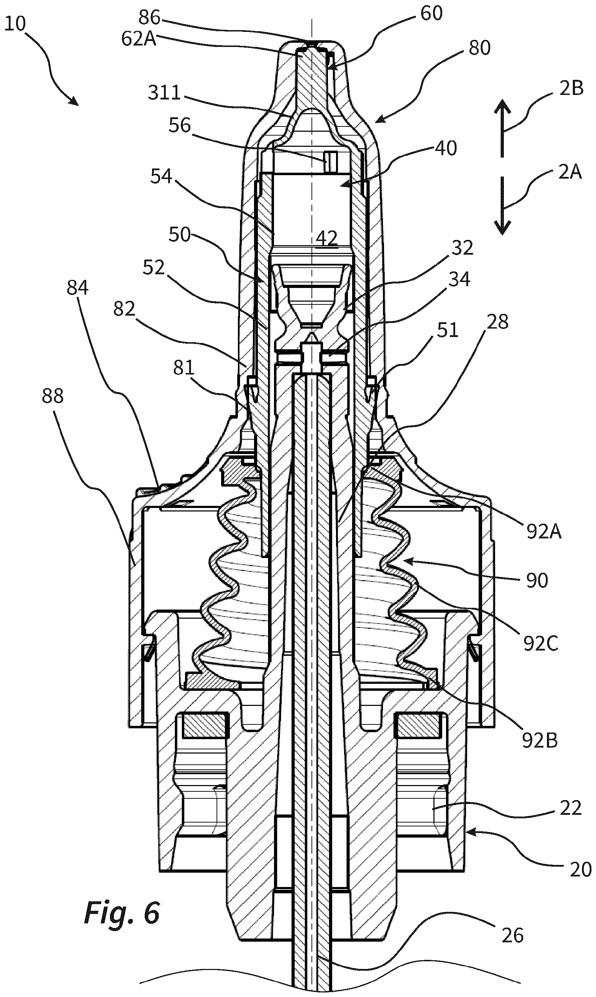

Figure 6 shows another embodiment of the invention. The discharge head 10 shown here has, like the previous embodiment, a discharge head base 20, on which a pump piston 32 is fixedly provided, a cylinder component 50 that is slid over the pump piston 32 and is movable relative to it, and an outer component 80, which forms a nasal applicator 82 and an actuating surface 84. A return spring 90 in the form of a plastic bellows spring is also provided here, which is supported on the discharge head base 20 and the cylinder component 50.

Unlike the design of the previous figures, this embodiment provides that the cylinder component 50 is not fixedly connected to the outer component 80, but is instead movable relative to it. To achieve this, a sealing lip 51 is provided on the outside of the cylinder component 50, which seals against an inner sliding surface 81 of the outer component 80 and thus prevents liquid from escaping into the area of the return spring 90.

Since the entire cylinder component 50 is movable relative to the outer component 80, no separate valve spring corresponding to the valve membrane 62B is required. As soon as the pump piston 32 reaches the cylinder sliding surface 54 during actuation, this leads to an increase in the liquid pressure in the pump chamber 42 and the valve chamber 64. This causes the cylinder component 50 to be displaced further downward in the actuating direction 2A than the outer component 80, so that the outlet valve 60 opens and the discharge begins.

When the discharge actuation is completed and the pump piston 32 has reached the upper end of the pump chamber 42, the operator reduces or completely releases the actuating force, so that the pressure in the pump chamber 42 dissipates. The return spring 90 then initially presses the valve body 62A into its closed position. Subsequently, the outer component 80 and the cylinder component 50 return to their starting positions shown in Figure 6.

A special feature of the design in Figure 6 is that despite the fact that no relative movement of the valve body 62A relative to the cylinder component 50 is required to open and close the valve, the valve body is attached to the cylinder component 50 by a spring section 311 and therefore has a certain degree of relative movability.

This relative movability is advantageous because it reduces the risk of the valve surface on the valve body 62A being pressed against the distal inner end of the outer component 80 with too much force, potentially causing damage. If the restoring force of the return spring 90 is too high, this is compensated by the spring section 311. This allows a relatively stiff bellows spring to be used as the return spring 90 without damaging the outlet valve 60. This delays the relaxation effect.

Despite the relative movability of the valve body 62A relative to the cylinder component 50, they are integrally formed. The spring section 311 designed to be deformable as intended is formed by an integrally molded bell-shaped membrane at the distal end of the cylinder component 50 and supports an integrally molded valve body 62A. The aforementioned sealing lip 51 is also an integral part of the cylinder component.

To make this cylinder component integrally, so that it allows both the desired deformability in the area of the spring section 311 and the sealing lip 51, while maintaining the required dimensional stability in the area of the cylinder sliding surface 54, LDPE or LLDPE is preferably chosen as the material. However, the sleeve section forming the cylinder sliding surface 54 is provided with a wall thickness that is at least twice as large as the wall thickness in the area of the spring section 311 and the sealing lip 51.

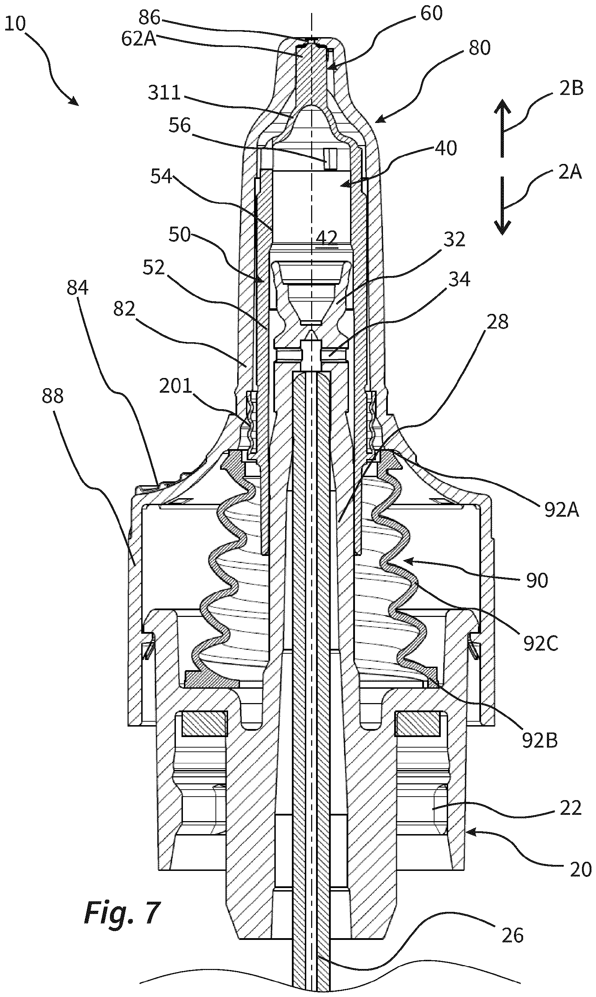

Figure 7 shows another embodiment. In accordance with the embodiment of Figure 6, it is also provided here that the cylinder component 50 is movable relative to the outer component 80, allowing the outlet valve 60 to be opened through this movement.

The special feature provided here is that an additional spring element 201 acts between the outer component 80 and the cylinder component 50. This spring element 201 is also designed as a bellows spring, but as a bellows tension spring. The upper end is fixed to the inner side of the outer component 80, for example, by snapping. The lower end of the spring element 201 is attached to the cylinder component, preferably by integral molding with the cylinder component.

Additionally, it is provided here that the return spring 90 is not acting on the cylinder component 50, but directly on the outer component 80.

If the actuating surface 84 and thus the outer component 80 is depressed, pressure builds up in the pump chamber 42 as described. Under the influence of this pressure, the cylinder component 50 is then pressed further downward than the outer component 80, so that the outlet valve 60 opens.

After the actuation is completed, the spring element 201 ensures that the outlet valve 60 closes immediately, regardless of the restoring force provided by the return spring 90.

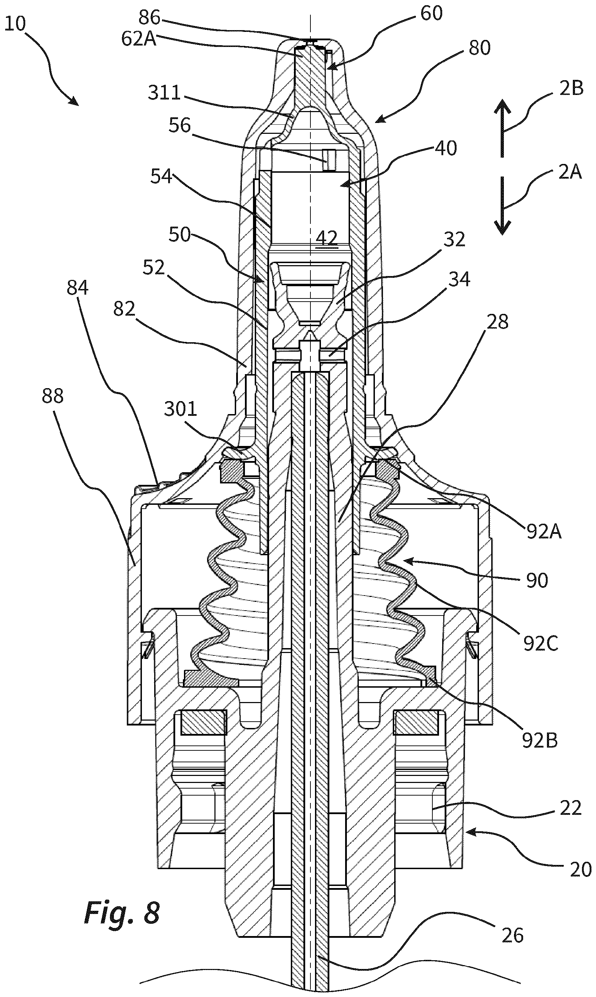

Figure 8 shows a second variant, which has a spring element 201 acting between the cylinder component 50 and the outer component 80. In this design, however, the spring element 201 is designed as a spring washer, which is clamped externally between the upper end 92A of the return spring 90 and an inner side of the outer component 80. The inner side of the spring washer ins integrally formed with the cylinder component 50.

All discharge heads described here consist exclusively of polyethylene. For the non-elastic components, HDPE is particularly suitable. For the described components 90, 61, and possibly also 50, the use of LDPE or LLDPE is particularly advantageous.

## Claims

1. Discharge head (10) for dispensing liquids, particularly pharmaceutical liquids, comprising the following features:
a. the discharge head (10) is designed to be attached to a liquid reservoir (100) and has a discharge head base (20) with a coupling device (22) for connecting to the liquid reservoir (100); and
b. the discharge head (10) has a discharge opening (86) and a pump device (40) for conveying liquid from the liquid reservoir (100) to the discharge opening (86); and
c. the pump device (40) includes a pump cylinder and a pump piston (32) that is at least temporarily guided in a sealing manner along a cylinder sliding surface (54); and
d. the pump piston (32) is fixedly arranged to the discharge head base (20); and
e. the pump cylinder is formed by a cylinder component (50) that is movable relative to the discharge head base (20);
**characterized by** the following feature:
f. a return spring (90) designed as a plastic bellows spring is provided, which exerts a counteracting return force on the cylinder component (50) when it is moved in an actuating direction (2A) relative to the pump piston (32).

2. Discharge head (10) according to claim 1, further comprising the following features:
a. the return spring (90) has bellows windings (92C) that decrease in diameter from a wide end of the return spring (90) towards a narrower end of the return spring (90); and
b. the wide end of the bellows spring forms a base end (92B) and is supported, at least indirectly, on the discharge head base (20); and
c. the narrow end of the bellows spring forms an applicator end (92A) and is supported, at least indirectly, on the cylinder component (50);
preferably with at least one of the following additional features:
d. the discharge head (10) has an actuating surface (84), wherein the wide end of the bellows spring extends into a receiving space located below the actuating surface (84) relative to an actuating direction (2); and/or
e. the discharge head (10) has a slim, elongated nasal applicator (82), whose outer diameter is radially exceeded by the base end (92B) of the return spring (90).

3. Discharge head (10) according to claim 1 or 2, further comprising the following feature:
a. an inner side of the cylinder component (50) has at least one section (52) where there is no circumferential seal between the pump piston (32) and the inner side of the cylinder component (50), with the pump piston (32) beingarranged in this non-sealing section (52) when the discharge head (10) is in an unactuated state.

4. Discharge head (10) according to any of claims 1 to 3, further comprising the following features:
a. the return spring (90) is designed as an internal component and is not visible to a user of the discharge head; and/or
b. an interior space of the return spring (90) is isolated from the surrounding environment.

5. Discharge head (10) according to any of claims 1 to 4, further comprising the following features:
a. the discharge head (10) has an outer component (80) which defines an applicator (82); and
b. the applicator (82) has an interior into which the cylinder component (50) is inserted; and
c. a distal end of the cylinder component (50) carries a valve body (62A), which, together with a valve surface provided on an inner side of the outer component (80), defines an outlet valve (60);
preferably with the following additional features:
d. on the inner side of the outer component (80), guide surfaces (89) are provided for guiding the valve body (62A); and/or
e. an actuating surface is provided on the outer component (80), particularly a ring-shaped actuating surface surrounding a slim, elongated applicator (84).

6. Discharge head (10) according to claim 5, further comprising the following features:
a. the outer component (80) and the cylinder component (50) are immovable relative to each other; and
b. at the distal end of the cylinder component (50), an elastically deformable valve membrane (62B) is attached or provided, which supports the valve body (62A) and is pulled into an open valve position by liquid pressure in a valve chamber (64);
preferably with the following additional feature:
c. the outlet valve (60) has a sealed counter chamber (66) on the side of the valve membrane (62B) opposite the valve chamber (64), with the counter chamber being at least partially bounded by the cylinder component (50).

7. Discharge head (10) according to claim 6, further comprising the following features:
a. a limiter surface is provided to limit the displacement of the valve body (62A) from the closed valve position to the open valve position; and
b. the limiter surface is formed by an end face of a limiting pin (58) provided at the distal end of the cylinder component (50).

8. Discharge head (10) according to claim 6 or 7, further comprising the following features:
a. a valve component is provided which integrally forms the valve body (62A) and the valve membrane (62B); and
b. the valve component has a sealing collar (62C) by which it is fitted onto a mounting surface of the cylinder component (50);
preferably with at least one of the following additional features:
c. the valve component has outwardly extending centering fins (62D), by which it is guided in the outer component (80) during assembly; and/or
d. a cross-section through the valve membrane shows at least two directional changes between the sealing collar (62C) and the valve body (62A), with the cross-section of the valve membrane preferably having the shape of an elongated "S".

9. Discharge head according to claim 5, further comprising the following features:
a. the cylinder component (50) is designed to be movable relative to the outer component (80); and
b. an outlet valve (160) with a valve body (162) is provided, which is opened and closed by the movement of the cylinder component (50) relative to the outer component.

10. Discharge head according to claim 9, further comprising the following feature:
a. the applicator end (92A) of the return spring (90) is supported on the cylinder component (50) and/or the outer component (80).

11. Discharge head according to claim 9 or 10, further comprising the following features:
a. a valve body (62A) is provided at the distal end of the cylinder component (50), which remains fixed relative to the cylinder sliding surface (52) of the cylinder component (50); or
b. a valve body (62A) is provided at the distal end of the cylinder component (50), which is formed with limited movability relative to the cylinder component (50) by a spring section (311).

12. Discharge head according to any of claims 9 to 11, further comprising the following feature:
a. a spring element (201, 301) is provided by which the outer component (80) and the cylinder component (50) are subjected to a closing force that acts in the direction of moving the valve body (162) into a closed valve position,
preferably with at least one of the following additional features:
b. the spring element (201) is designed as a bellows spring; or
c. the spring element (301) is designed as a spring washer that is internally fixed to the cylinder component and externally supported on the outer component (80).

13. Discharge head according to any of claims 6 to 8, 11, or 12, further comprising the following feature:
a. the spring element (201, 301), the spring section (311), or the valve membrane is integrally formed with the cylinder component (50),
preferably with at least one of the following features:
b. the spring element (201, 301) has a wall thickness that is at most 50% of the wall thickness of the cylinder component (50) in the area of the cylinder sliding surface, preferably at most 20%; and/or
c. the spring section (311) has a wall thickness that is at most 50% of the wall thickness of the cylinder component (50) in the area of the cylinder sliding surface, preferably at most 20%; and/or
d. the valve membrane has a wall thickness that is at most 50% of the wall thickness of the cylinder component in the area of the cylinder sliding surface, preferably at most 20%.

14. Discharge head according to any of the preceding claims, further comprising the following feature:
a. the discharge head is made exclusively from materials that can be recycled together in a common recycling process,
preferably with the following additional feature:
b. the discharge head is made exclusively of polyethylene (PE).

15. Liquid dispenser comprising the following features:
a. the liquid dispenser has a liquid reservoir (100); and
b. the liquid dispenser has a discharge head (10),
**characterized by** the following feature:
c. the discharge head (10) is designed according to any of the preceding claims.
